# EUROPEAN PATENT APPLICATION

(11) **EP 1 059 072 A2**
(43) Date of publication of application: **13.12.2000**
(21) Application number: 00304639.8
(22) Date of filing: 31.05.2000
(51) Int. Cl.: A61F 13/15

(54) **Disposable diaper having a function of preventing back leakage of feces**

(30) Priority: 07.06.1999 JP 15965999
(71) Applicant: UNI-CHARM CORPORATION, Kawanoe-shi Ehime-ken (JP)
(72) Inventor: Ohashi, Naoto, c/o Technical Center, Mitoyo-gun, Kagawa-ken 769-1602 (JP)
(74) Representative: Parry, Christopher Stephen

(57) **Abstract**

There is disclosed a disposable diaper having a front part for fitting a belly, an intermediate part for fitting a crotch and a back part for fitting a back of a wearer in order along a lengthwise direction of the diaper. The diaper includes a liquid pervious top sheet for facing a wearer, a back sheet for facing outside and an absorbent core put between the top sheet and the back sheet. At least in the back part and along a lengthwise extending centerline of the diaper, there is disposed a thick portion rising toward the wearer while gradually decreasing in its size in a width direction perpendicular to the lengthwise direction to have a crest. The width size of the thick portion at a bottom thereof is smaller than the width size of the absorbent core at the intermediate portion.

## Description

### BACKGROUND OF THE INVENTION

### Field of the invention

The present invention concerns a disposable diaper having a function of preventing back leakage and, more in particular, it relates a disposable diaper improved for the function of preventing leakage from a back part.

### Description of the Related Art

In recent years, open type disposable diapers of an hourglass shape have been used generally.

The diaper comprises a liquid pervious top sheet facing a wearer, a liquid impervious back sheet facing outside and an absorbent core put between the top sheet and the back sheet and it includes a front part facing a belly, an intermediate part facing a crotch and a back part facing a back of a wearer.

However, since the open type diaper has a planer shape, it can not be fitted closely with a sterical buttock, particularly, with a natal cleft. Then, such an open type diaper is put on by a baby or an infant usually in a lying state. Accordingly, when the wearer discharges, for example, soft feces in the lying state, it results in a problem that the soft feces leak from the back waist part to the outside through gap between the diaper and the natal cleft.

### SUMMARY OF THE INVENTION

An object of the present invention is to provide a disposable diaper having a function of preventing leakage of feces from a back waist part of the diaper to the back of the wearer, that is, a function of preventing back leakage.

The present invention provides a disposable diaper having a front part for fitting a belly, an intermediate part for fitting a crotch and a back part for fitting a back of a wearer in order along a lengthwise direction of the diaper, comprising a liquid pervious top sheet for facing a wearer, a back sheet for facing outside and an absorbent core put between the top sheet and the back sheet, wherein
at least in the back part and along a lengthwise extending centerline of the diaper, there is disposed a thick portion rising toward the wearer while gradually decreasing in its size in a width direction perpendicular to the lengthwise direction to have a crest, and the width size of the thick portion at a bottom thereof is smaller than the width size of the absorbent core at the intermediate part.

### BRIEF DESCRIPTION OF THE DRAWINGS

Fig. 1 is a plan view showing a disposable diaper on the side of a liquid pervious sheet according to one embodiment of the present invention;
Fig. 2 is a cross sectional view taken along line II-II of the diaper shown in Fig. 1;
Fig. 3 is a cross sectional view taken along line III-III of the diaper shown in Fig 1;
Fig. 4 is a cross sectional view for a thick portion in the direction X in Fig. 1;
Fig. 5 is a modified embodiment for the thick portion shown in Fig. 4;
Fig. 6 is an explanatory view showing a method of manufacturing the disposable diaper shown in Fig. 1;
Fig. 7 is a perspective view of a patterning drum shown in Fig. 6;
Fig. 8 is an enlarged cross sectional view for a portion of the patterning drum shown in Fig. 6;
Fig. 9 is a perspective view showing another example of a thick portion;
Fig. 10 is a cross sectional view taken along line I0-I0 in Fig. 9;
Fig. 11 is a perspective view showing another example of a thick portion;
Fig. 12 is a cross sectional view taken along line I2-I2 in Fig. 11;
Fig. 13 is a perspective view for explaining a further example of a thick portion;
Fig. 14 is a plan view showing a disposable diaper according to another embodiment of the invention; and
Fig. 15 is a fragmentary cross sectional view for a thick portion in the direction X in Fig. 14.

### DETAILED DESCRIPTION OF THE INVENTION

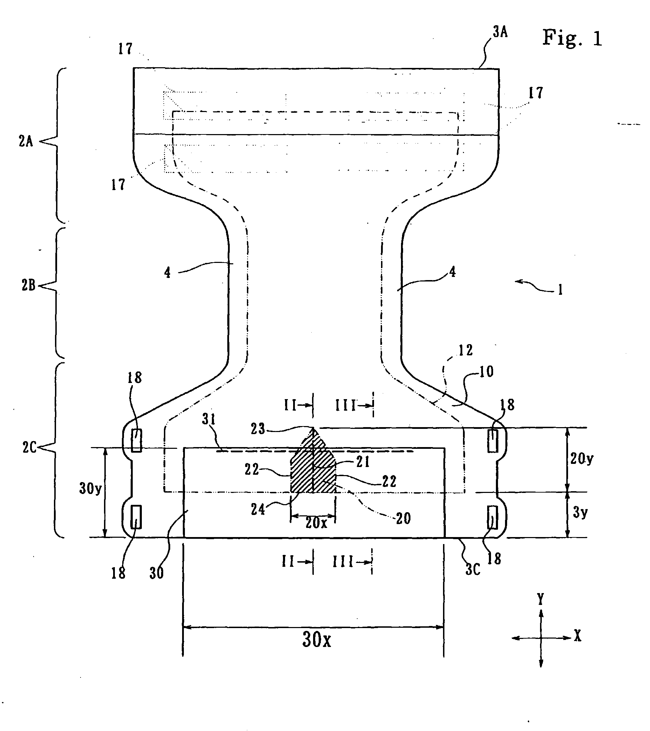

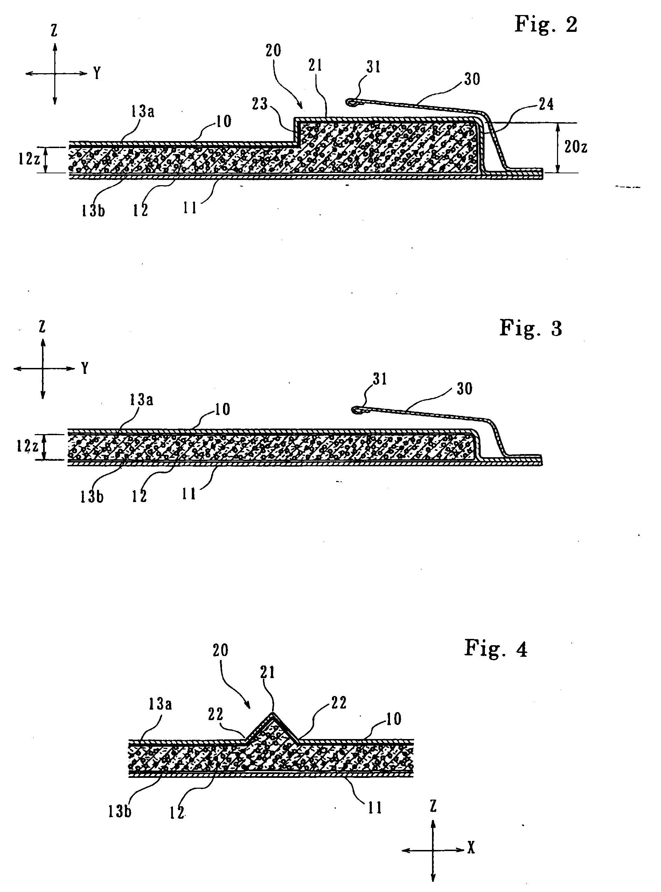

A disposable diaper according to the present invention is to be explained with reference to the drawings. Fig. 1 is a plan view showing a disposable diaper on the side of a liquid pervious sheet according to one embodiment of the present invention, Fig. 2 is a cross sectional view taken along line II-II of the diaper shown in Fig. 1, Fig. 3 is a cross sectional view taken along line III-III of the diaper shown in Fig. 1 and Fig. 4 is a cross sectional view for a thick portion in the direction X in Fig. 1.

As shown in Fig. 1, a disposable diaper 1 according to one embodiment of the present invention is an open type diaper having a so-called hourglass shape and includes a front part 2A facing a belly in use, a back part 2C facing a buttock and/or back in use and an intermediate part 2B facing a crotch in use of a wearer. It is assumed here that a direction from the front part by way of the intermediate part to the back part is direction Y (lengthwise direction) and a direction perpendicular thereto is a direction X (width direction). Further, as shown in Figs. 2, 3 and 4, the direction toward the wearer is assumed as direction Z.

The disposable diaper 1 comprises a liquid pervious top sheet 10 facing the wearer, a back sheet 11 directed to the outside and an absorbent core 12 put between the top sheet 10 and back sheet 11. In the embodiment shown, each of the top sheet 10, the back sheet 11 and the adsorbent core 12 is in an hourglass shape. The top sheet 10 and the back sheet 11 are bonded to each other, for example, with a hot melt type adhesive at the periphery of the absorbent core 12.

Upon putting, the disposable diaper 1 is retained and secured to a wearer's body at the periphery of a waist. In the embodiment shown, back flaps of the back part 2C (portions protruding in the direction X) are overlapped on the back sheet 11 of the front part 2A, and engagement sheets 18 disposed on both edges of the top sheet 10 of the back flaps of the back part 2C and engagements 17 disposed to the front flap (portions protruding in the direction X) of the back sheet 11 of the front part 2A are engaged at the waist of the wearer.

The top sheet 10 may be formed of any suitable material, for example, hydrophilized hydrophobic fibers or hydrophilic fibers. The term "hydrophilized hydrophobic fiber" as used herein means a hydrophobic fiber subjected to a hydrophilic treatment. In this hydrophilic treatment, a hydrophobic fiber is made hydrophilic, for example, by treating it with a surfactant; by chemically binding a chemical substance such as a monomer or a polymer having a hydrophilic group thereto; by subjecting it to plasma processing; by kneading it with a chemical substance having a hydrophilic group; or by treating its surface to have a profiled section. The top sheet 10 comprises, for example, point bond, air though, spun bond or spun lace non-woven fabric. Alternatively, the top sheet may be formed by overlapping a cushioning layer comprising a bulky non-woven fabric on the non-woven fabric described above. In the embodiment shown, the back sheet 11 is liquid impervious but air permeable and is formed, for example, of a polyolefinic resin sheet. Alternatively, it is also possible that the back sheet is made of a non-woven fabric while a water proof film is interposed between the back sheet and the absorbent core. In the case where the diaper is used being stacked on another absorbent article, moreover, the back sheet may be liquid pervious.

The absorbent core 12 may be formed of any suitable absorbent material, for example, pulverized pulp or a mixture of pulverized pulp and highly absorbent polymer. The highly absorbent polymer can be made of polyacrylic acid, sodium polyacrylate, polyacrylamide, polyacrylonitrile, polyvinyl alcohol, an additional polymer of maleic anhydride, a polyether, a condensed polymer, a polysaccharide such as starch or cellulose, a protein such as collagen, or the like. Examples of the highly absorbent polymers include: a cross-linked compound of sodium polyacrylate, a graft copolymer of starch having sodium polyacrylate or a graft copolymer of cellulose having polyacrylonitrile chains. In the embodiment shown, the absorbent core 12 is formed by wrapping a mixture of pulverized pulp and highly absorbent polymer with an absorbing sheet 13 such as tissue paper.

The engagement sheet 18 is, for example, a resin sheet having a plurality of mushroom-shaped or hook-shaped engagement protrusions each having an engaging head at the top end and the engagement 17 is, for example, a tricot-knitted woven fabric or non-woven fabric using polyester fibers as a material. Alternatively, the engagement 17 may be an adhesive tape made of rubber adhesive material or acrylic resin, and the engagement sheet 18 may be a resin film.

In the back part 2C of the disposable diaper 1, there is formed a thick portion 20 on the side of a wearer. When the thick portion is positioned only in the back part, discharges are well dammed up by the thick portion. In this embodiment, as shown in Fig. 1, the thick portion 20 has a substantially pentagonal planer shape, with a top end 23 being directed to the intermediate part 2B and a base end 24 being directed to the back waist part 3C. That is, the width size (i.e., the size in the direction X) of a bottom 22 of the thick portion 20 is larger at the base end 24 and gradually decreased toward the top end 23. Further, as shown in the cross section along the direction X of the thick portion 20 in Fig. 4, the width size of the thick portion 20 is gradually decreased from the bottom 22 to a crest 21 to form a substantially trigonal transversal cross section. The thick portion 20 has such a substantially trigonal shape at any transversal cross section except for the top end 23 of the pentagonal shape on the side of the intermediate part 2B. That is, the crest 21 is present for the entire length along the direction Y in the thick portion 20.

Thus, the thick portion 20 has such a shape as can intrude into a natal cleft of a wearer. Further, since the top end 23 has a tapered shape, it gives less feel of incongruity when intrudes into the natal cleft. Because the thick portion 20 is present at the back part 2C, even when discharges such as soft feces that are less absorbed by the absorbent core 12 should flow toward the back part, they are dammed up by the thick portion 20 and the discharges do not leak from the back waist part 3C to the outside of the diaper.

In the embodiment shown in Fig. 1, the bottom 22 of the thick portion 20 has a width size 20x in the direction X of about 20 to 40 mm, more preferably, about 25 to 35 mm. The size 20y in the direction Y is about 50 to 70 mm, more preferably, about 55 to 65 mm. Further, the distance 3y between the base end 24 of the thick portion 20 and the back waist part 3C is about 30 to 50 mm, and more preferably, about 35 to 45 mm. Here, the back waist part 3C indicates the end edge of the diaper 1, on the side of the back part 2C, as shown in Fig. 1.

The thick portion 20 preferably comprises a liquid absorbent such that this portion itself can absorb discharges. The liquid absorbent of the thick portion 20 is covered with the top sheet 10 like that the absorbent core 12 in other areas.

For example, as shown in Fig. 2 and Fig. 4, the liquid absorbent of the thick portion 20 can be formed integrally with the absorbent tore 12. That is, the liquid absorbent of the thick portion 20 can be formed by increasing the basis weight in a certain portion of the absorbent core 12. For instance, in this case, the absorbent core has a thickness 20z of 4.5 mm at the thick portion 20 and has a thickness 12z of 2.5 mm in other area.

For allowing the thick portion 20 to intrude into the natal cleft of the wearer thereby reducing the gap between the diaper 1 and the natal cleft, the thick portion 20 is preferably raised from the surface of the absorbent core 12 by 2 mm or more. In other words, the difference between the thickness 20z and the thickness 12z is preferably 2 mm or more.

In a case where the liquid absorbent of the thick portion 20 is formed integrally with the absorbent core 12, more absorbent material is present at a location formed with the thick portion 20. For example, the absorbent material (for example, pulp) of the absorbent core 12 has a basis weight of about 250 g/m² at a location where the thick portion 20 is not present, while it has a basis weight of about 400 g/m at a location where the thick portion 20 is formed. Accordingly, since the absorption at the thick portion 20 is increased and the water content of discharges such as soft feces is absorbed more effectively, the leakage of the discharges can be further prevented.

Further, in the disposable diaper 1, a cuff (sterical gather) 30 for preventing leakage at the waist may be disposed. For example, as shown in Fig. 1, a liquid impervious stripe sheet is disposed between the thick portion 20 and the back waist part 3C, and an elastic member 31 is disposed on one side edge of the stripe sheet on the side of the intermediate part 2B. The stripe sheet is joined to the top sheet 10 at both ends in the direction X and at the side edge on the side of the back waist part 3C to form an upstanding base (base end). As a result, the cuff 30 for preventing waist leakage open to the intermediate part 2B is formed. The cuff 30 is disposed so as to extend to the surface of the thick portion 20 and cover the thick portion 20, and a pocket is formed between the slope of the thick portion 20 and the cuff 30. Accordingly, even when a great amount of discharges that can not be dammed up completely with the thick portion 20 are excreted and flow toward the back waist part 3C rounding about the thick portion 20, they are caused to enter and kept in the pocket to further suppress the leakage.

The liquid impervious stripe sheet forming the cuff 30 can be formed, for example, from a non-woven fabric made of hydrophobic fibers or a resin sheet. Preferably, the liquid impervious stripe sheet is an air permeable sheet which is formed, for example, from only a spun bond non-woven fabric of polypropylene fibers or by overlapping a spun bond non-woven fabric, melt blown non-woven fabric and spun bond non-woven fabric.

The size of the stripe sheet in the direction X is preferably from 170 to 130 mm and, more preferably, about 150 mm. The size of the cuff 30 in the direction Y from the back waist part 3C is, for example, from 70 to 50 mm and, preferably, about 60 mm. However, the waist-side edge of the stripe sheet is not necessarily stacked just on the back waist part 3C but may be disposed at a portion apart from the back waist part 3C to the intermediate part 2B in which the size of the stripe sheet in the direction Y can be varied properly.

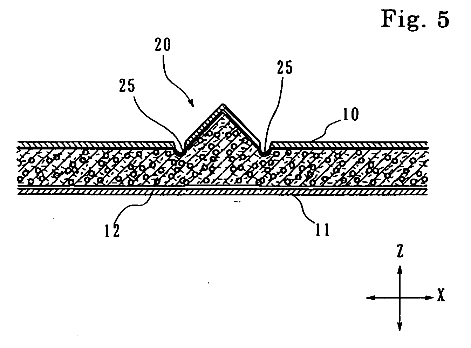

In a case of forming the cuff 30, a groove 25 may be formed at the periphery of the thick portion 20 as shown in Fig. 5. The groove 25 is formed along the periphery of the thick portion 20 except for the base end 24 on the side of the back waist part 3C. The groove 25 facilitates flow of the discharges along the groove and facilitates introduction of them into the pocket of the cuff 30. The groove is preferably formed by applying a hinge embossing or heat sealing to the absorbent core 12. In this case, the density of the absorbent material in the groove 25 is increased thereby facilitating introduction of the discharges to the groove 25 by capillary action.

Further, a side leakage preventive cuff adapted in existent open type diapers may be disposed to the diaper 1. For example, liquid impervious stripe sheets are disposed on both sides 4 and 4 on the top sheet 10. Each stripe sheet is provided with an elastic member, along one side edge directed to the center of the diaper 1 (or along its mid-portion between two side edges), and is then joined to the upper surface of the top sheet 10, at the opposite side edge and two end edges thereof. As a result, a pair of cuffs for preventing side leakage are formed such that the side edges directed to the center are raised upon putting of the disposable diaper.

Furthermore, so-called leg cuffs may also be provided. For example, in the region for both sides 4 and 4 of the disposable diaper 1 where the absorbent core 12 is not present (outside of the absorption region), elastic members extending in the lengthwise direction (direction Y) of the disposable diaper 1 are bonded and secured between the top sheet 10 and the back sheet 11. Elastic shrinkage of the elastic members in the direction Y causes the top sheet 10 and the back sheet 11 to shrink in the region for both sides 4 and 4 of the disposable diaper 1 and fit to the periphery of legs, to form leg gathers.

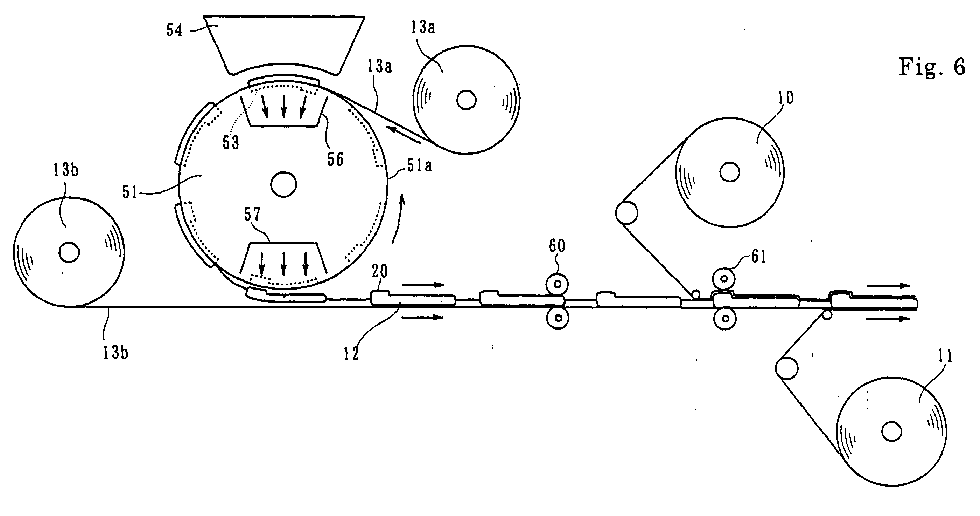

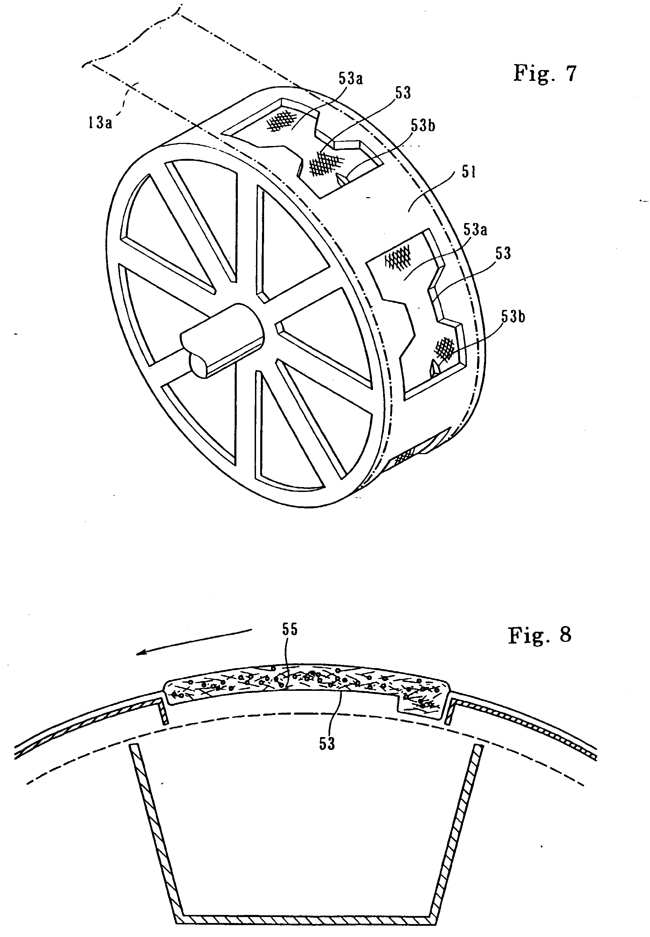

Then, an example of a manufacturing method for a disposable diaper 1 having the thick portion 20 is to be explained. However, the manufacturing method of the disposable diaper 1 is not restricted to this example. Fig. 6 is an explanatory view showing a method of manufacturing the disposable diaper shown in Fig. 1, Fig. 7 is a perspective view of a patterning drum shown in Fig. 6 and Fig. 8 is an enlarged cross sectional view for a portion of the patterning drum shown in Fig. 6.

At first, as shown in Fig. 6, an upper tissue 13a is continuously supplied from a raw fabric on the outer circumferential surface 51a of a patterning drum 51 that rotates at a constant speed in a counterclockwise direction. In the patterning drum 51, recesses 53 each of an hourglass shape as shown in Fig. 7 are formed on the outer circumferential surface 51a.

The bottom of the recess 53 is formed as a mesh 53a and a trigonal recess 53b for forming the thick portion 20 is formed in the mesh 53a. When the patterning drum 51 rotates and the recess 53 is situated at the uppermost position of the patterning drum 51 (that is, above a suction chamber 56), a suction pressure from the suction chamber 56 is exerted on the tissue 13a through the mesh 53a, so that the tissue 13a is placed along the inner surface at the inside of the recess 53 and deformed into a concave shape.

In this state, pulverized pulp and highly absorbent polymer are supplied from an absorbent material accumulation machine 54 into the recess 53 and the suction pressure from the suction chamber 56 is also exerted thereon. As a result, as shown in Fig. 8, the upper tissue 13a is laid on the surface of the mesh 53a in the recess 53 and an absorbent material layer 55 is formed on the tissue 13a. Thus, when the patterning drum 51 shown in Fig. 7 is used, the absorbent material layer 55 can be in the hourglass shape while having a portion of an increased thickness to form the thick portion subsequently.

On the other hand, a lower tissue 13b is delivered from a raw-fabric and sent out to a position below the patterning drum 51. When the recess 53 moves to the lowermost position of the patterning drum 51, the absorbent material layer 55 formed in the recess 53 and the upper tissue 13a are separated from the inside of the recess 53 by a pressurization chamber 57. Therefore, the absorbent material layer 55 is relocated together with the upper tissue 13a onto the lower tissue 13b to form the absorbent core 12. In this absorbent core 12, the thick portion 20 protrudes toward the upper tissue 13a.

Subsequently, the area of the absorbent core 12 except for the thick portion 20 is pressed by a first hinge embossment 60 to flatten the surface of the absorbent core 12. A top sheet 10 rolled as a raw fabric is supplied to the surface of the absorbent core 12 and laminated on the absorbent core 12. Then, both of the absorbent core 12 and the top sheet 10 are pressed by a second hinge embossment 61 to be shaped in the area other than the thick portion 20. In this way, an absorbent core 12 is formed, having the thickness in the direction Z of the thick portion 20 of about 4.5 mm and the thickness for other area of about 2.5 mm, and the rigidity for the area other than the thick portion 20 of from 10 to 30 g. Further, a back sheet 11 rolled as a raw fabric is supplied to and accumulated on the lower side (back side) of the laminate of the absorbent core 12 and the top sheet 10.

Subsequently, although not illustrated in Fig. 6, the top sheet 10 and the back sheet 11 are bonded by a hot melt adhesive or thermal fusion at the periphery of the absorbent core 12 to be integrated. The integrated piece is then cut by means of cutter or the like to obtain individual disposable diapers 1. Other steps such as for disposing side leakage preventive gathers, leg gathers and waist gathers may be optionally added although not described particularly here.

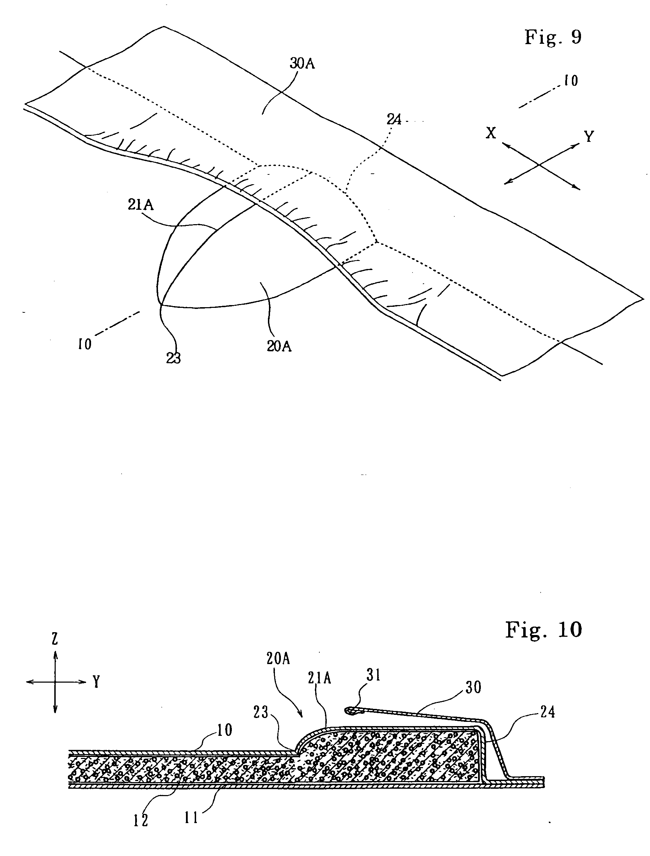

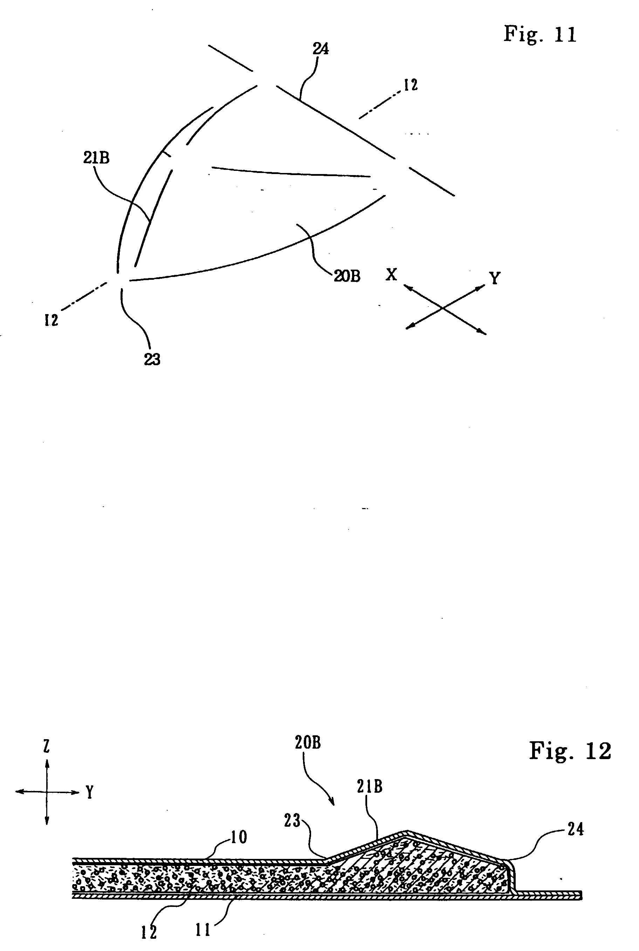

Fig. 9 and Fig. 11 are perspective views illustrating other examples of the thick portion respectively. Fig. 10 is a cross sectional view taken along line I0-I0 in Fig. 9 and Fig. 12 is a cross sectional view taken along line I2-I2 in Fig. 11.

As shown in Fig. 9 and Fig. 10, a thick portion 20A may be formed such that a crest 21A gradually approaches the absorbent core 12 as it extends to the top end 23, that is, the height size of the thick portion 20A is gradually lowered.

Further, as shown in Fig. 11 and Fig. 12, a thick portion 20B of a triangular pyramidal shape may be formed. In this case, a crest 21B gradually approaches the absorbent core 12 as it extends to the top end 23 and the height size of the thick portion 20B is gradually lowered. On the other hand, the crest 21B gradually approaches the absorbent core 12 as it extends to the base end 24 and the height size of the thick portion 20B is gradually lowered. Thus, as shown in Fig. 12, the crest 21B has the maximum height size about at the intermediate portion between the top end 23 and the base end 24. The maximum height size is preferably 2 mm or more.

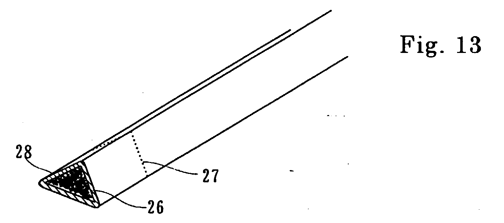

The method of forming the thick portion is not restricted only to the increase of the thickness of the absorbent core 12 in the direction Z. For example, an absorbent material having a shape of the thick portion shown in Fig. 9 or Fig. 11 may be covered with a non-woven fabric or the like and bonded and secured to the back part 2C of the top sheet 10. Further, as shown in Fig. 13, a tow 28 formed by bundling a plurality of long continuous fibers (filaments) in one identical direction into a trigonal post shape may be wrapped with a non-woven fabric 26, cut at a predetermined distance (for example, along a cut line 27) and put between the absorbent core 12 and the top sheet 10, to form the thick portion. Furthermore, the thick portion may be formed of a cushioning material such as a urethane foam material. It is, however, desirable that the thick portion is formed of an absorbent material so that discharges can be absorbed even at the thick portion.

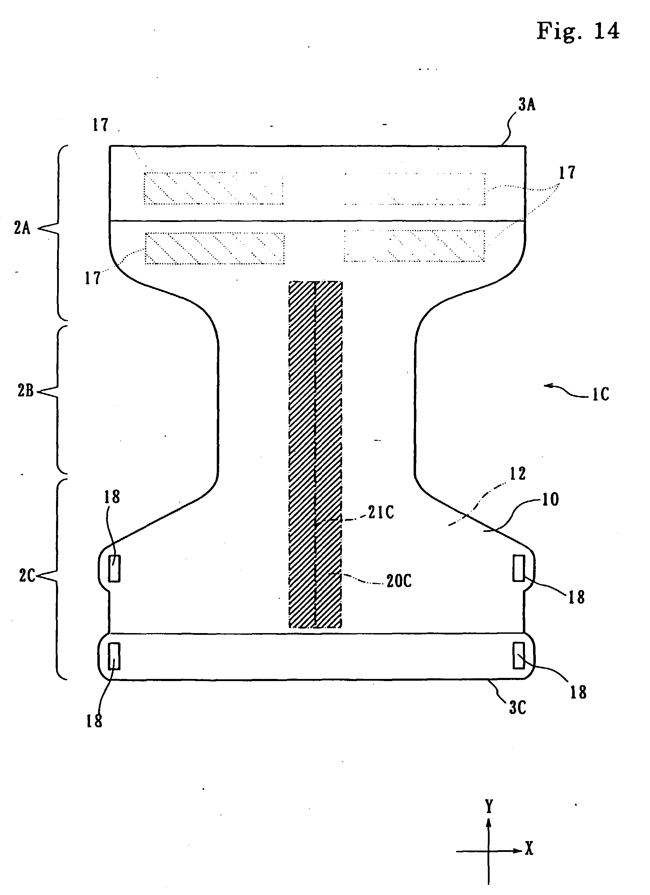

Fig. 14 is a plan view showing a disposable diaper 1C according to another embodiment of the invention. In Fig. 14, a thick portion 20C extends lengthwise and continuously from the front part 2A by way of the intermediate part 2B to the back part 2C. The cross sectional shape in the direction X is a trigonal shape at any position as shown in Fig. 4. When such a thick portion 20C is disposed, since there is no gap between the natal cleft and the disposable diaper 1C, discharges are not moved but absorbed in the absorbent core. Accordingly, even when soft feces are discharged, since the water content therein can be absorbed reliably in the absorbent core, the soft feces are less moved to the back waist part and leakage can be prevented.

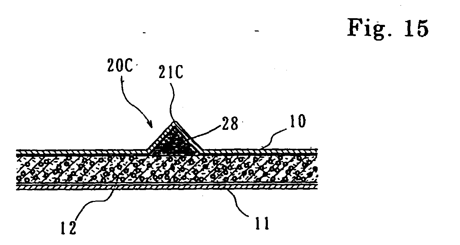

Such a thick portion 20C can be obtained by increasing the thickness of the absorbent core 12 in the direction Z like that the thick portion 20 shown in Fig. 1. In addition, the thick portion 20C can also be formed by disposing the tow 28 between the absorbent core 12 and the top sheets 10 as shown in Fig. 15. Further, although not illustrated in Fig. 14, the cuff for preventing leakage at the waist as shown in Fig. 1 is preferably disposed also in this diaper.

The disposable diaper of the invention is not restricted to the open type having an hourglass shape but it may be a rectangular open type or a disposable diaper previously formed into a pant type.

Furthermore, means for retaining the diaper to a wearer's body is not restricted to the engagement in the waist part but the diaper may be retained by other means (for example, suspender).

As has been described above specifically, in the diaper disposed with the thick portion of the invention, no gap is formed between the diaper and the natal cleft of a wearer. Accordingly, even when a lying wearer discharges soft feces, leakage of discharges from the back waist part of the diaper to the back of the wearer (to the outside of the diaper) can be prevented by damming up them with the thick portion.

Alternatively, the disposable diaper of the invention is well fitted in a crotch part and excellent in feeling upon wearing.

Here, 'comprises/comprising' when used in this specification is taken to specify the presence of stated features, integers, steps or components but does not preclude the presence or addition of one or more other features, integers, steps, components or groups thereof.

Although various exemplary embodiments have been shown and described, the invention is not limited to the embodiments shown. Therefore, the scope of the invention is intended to be limited solely by the scope of the claims that follow.

## Claims

1. A disposable diaper having a front part for fitting a belly, an intermediate part for fitting a crotch and a back part for fitting a back of a wearer in order along a lengthwise direction of the diaper, comprising a liquid pervious top sheet for facing a wearer, a back sheet for facing outside and an absorbent core put between the top sheet and the back sheet, wherein
at least in the back part and along a lengthwise extending centerline of the diaper, there is disposed a thick portion rising toward the wearer while gradually decreasing in its size in a width direction perpendicular to the lengthwise direction to have a crest, and the width size of the thick portion at a bottom thereof is smaller than the width size of the absorbent core at the intermediate part.

2. The disposable diaper as defined in claim 1, wherein the thick portion is situated in the back part and has a top end directed to the intermediate part and a base end directed oppositely, and the width size of the thick portion at the bottom is larger on the side of the base end and is gradually decreased toward the top end.

3. The disposable diaper as defined in claim 2, wherein the height size of the thick portion is gradually lowered toward the top end.

4. The disposable diaper as defined in claim 1, wherein the thick portion is formed by covering the surface of a liquid absorbent with the top sheet.

5. The disposable diaper as defined in claim 4, wherein the liquid absorbent of the thick portion is formed continuously with the material identical with the absorbent core, so that the basis weight of the absorbent core including the thick portion at an area where the thick portion is disposed is greater than the basis weight of the absorbent core in the area other than the area disposed with the thick portion.

6. The disposable diaper as defined in claim 5, wherein the thick portion is raised from the surface of the absorbent core by 2 mm or more.

7. The disposable diaper as defined in claim 5, wherein the back part is provided with a sterical gather starting outside of the thick portion and extending over the thick portion for preventing lengthwise leakage of feces from the back part.

8. The disposable diaper as defined in claim 7, wherein a groove is formed along the periphery of the bottom of the thick portion.

9. The disposable diaper as defined in claim 8, wherein the groove is formed by applying heat embossing or heat sealing to the absorbent core.

10. The disposable diaper as defined in claim 2, wherein the thick portion is formed by covering the surface of a liquid absorbent with the top sheet.

11. The disposable diaper as defined in claim 10, wherein the liquid absorbent of the thick portion is formed continuously with the material identical with the absorbent core, so that the basis weight of the absorbent core including the thick portion at an area where the thick portion is disposed is greater than the basis weight of the absorbent core in the area other than the area disposed with the thick portion.

12. The disposable diaper as defined in claim 11, wherein the thick portion is raised from the surface of the absorbent core by 2 mm or more.

13. The disposable diaper as defined in claim 11, wherein the back part is provided with a sterical gather starting outside of the thick portion and extending over the thick portion for preventing lengthwise leakage of feces from the back part.

14. The disposable diaper as defined in claim 13, wherein a groove is formed along the periphery of the bottom of the thick portion.

15. The disposable diaper as defined in claim 14, wherein the groove is formed by applying heat embossing or heat sealing to the absorbent core.

16. The disposable diaper as defined in claim 1, wherein the back part is provided with a sterical gather starting outside of the thick portion and extending over the thick portion for preventing lengthwise leakage of feces from the back part.

17. The disposable diaper as defined in claim 16, wherein a groove is formed along the periphery of the bottom of the thick portion.

18. The disposable diaper as defined in claim 17, wherein the groove is formed by applying heat embossing or heat sealing to the absorbent core.

19. The disposable diaper as defined in claim 1, wherein the thick portion is raised from the surface of the absorbent core by 2 mm or more.
